Europäisches Patentamt

**European Patent Office**

Office européen des brevets

(19)

(11) Numéro de publication: **0 125 169**
**B1**

(12) **FASCICULE DE BREVET EUROPEEN**

(45) Date de publication du fascicule du brevet: **07.10.87**

(21) Numéro de dépôt: **84400859.9**

(22) Date de dépôt: **27.04.84**

(51) Int. Cl.⁴: **C 07 C 119/048,**
**C 07 C 118/02, C 07 C 118/00**

(54) **Composition liquide, contenant des diisocyanates à structure diphénylméthane, et son procédé de fabrication.**

(30) Priorité: **29.04.83 FR 8307116**

(43) Date de publication de la demande:
**14.11.84 Bulletin 84/46**

(45) Mention de la délivrance du brevet:
**07.10.87 Bulletin 87/41**

(84) Etats contractants désignés:
**BE CH DE FR GB IT LI NL**

(56) Documents cités:
**EP-A-0 024 665**
**EP-A-0 046 556**
**EP-A-0 057 862**
**EP-A-0 092 454**
**DE-A-2 930 411**
**FR-A-2 380 309**
**FR-A-2 382 429**
**GB-A-1 398 975**

(73) Titulaire: **ATOCHEM**
**4 & 8, Cours Michelet La Défense 10**
**F-92800 Puteaux (FR)**

(72) Inventeur: **Kervennal, Jacques**
**134, Rue E. Locard**
**F-69005 Lyon (FR)**
Inventeur: **Mathais, Henri Hameau de St-Didier**
**Villa no 2 Chemin de l'Indiennerie**
**F-69370 Saint-Didier-au-Mont-d'Or (FR)**
Inventeur: **Commandeur, Raymond**
**Le Rocher Avenue de Venaria**
**F-38220 Vizille (FR)**

(74) Mandataire: **Foiret, Claude et al**
**ATOCHEM Département Propriété Industrielle**
**F-92091 Paris la Défense 10 Cédex 42 (FR)**

EP 0 125 169 B1

Courier Press, Leamington Spa, England.

**Description**

L'invention concerne une nouvelle composition liquide, contenant des diisocyanates à structure diphénylméthane, obtenue par réaction initiale d'un halogénure de benzyle sur le benzène, suivie d'une nitration des noyaux aromatiques, d'une hydrogénation et d'une phosgénation. La répartition en poids des diisocyanates dans cette composition est de 32 à 55% d'isomère 4,4', de 30 à 45% d'isomère 2,4', de 5 à 12% d'isomère 3,4', de 4 à 10% d'isomère 2,2', de 2 à 5% d'isomère 2,3': elle est utilisable dans la fabrication de polyuréthannes.

Des diisocyanates de structure aromatique hydrocarbonée diphénylméthane (M.D.I.) sont déjà synthétisés industriellement; ils sont obtenus par condensation de deux molécules d'aniline sur une de formol en présence d'un catalyseur acide, suivie d'une phosgénation, ce qui conduit à des mélange contenant les isomères 4,4', 2,4' et 2,2'. Mais cette condensation n'est jamais totalement sélective en produits à 2 noyaux et on synthétise habituellement un mélange de polyisocyanates et de diisocyanates, ces derniers étant séparés par distillation pour donner du MDI pur. L'isomère MDI 4,4' est en général nettement majoritaire et possède un point de fusion de 38°C; ainsi, suivant sa teneur il peut être nécessaire de liquéfier le mélange de diisocyanates avant de pouvoir l'utiliser, soit en le fondant (DE—A—2 930 411), soit en le transformant chimiquement (FR—A—2 380 309; EP—A—0 057 862). De plus, de tels mélanges solides supportent mal le stockage et doivent être conservés en chambre froide.

Les mélanges conformes à l'invention présentent l'avantage d'être liquides à température ordinaire, ce qui les rend directement utilisables, tout en étant constitués uniquement d'isocyanates; de plus, ils se conservent bien. En outre, la partie benzylique du système diphénylméthane provient de toluène, matière première moins coûteuse que le benzène, ce qui représente un gain économique sensible.

Par le EP—A—0 024 665 est connu un mélange de diisocyanates à structure diphénylméthane dont l'un des noyaux aromatiques est éventuellement porteur d'un groupement méthyle, Ledit mélange comporte au moins 30% d'isomère 3,4', jusqu'à 40% d'isomère 2,3' et jusqu'à 30% d'autres isomères. Ledit mélange est obtenu par nitration d'un composé à structure diphénylméthane suivie d'une hydrogénation et d'une phosgénation. Cependant, ou bien ledit composé est obtenu par réaction de nitrohalogénure de benzyle, éventuellement substitué par un groupement méthyle, sur du nitrobenzène, du benzène ou du toluène et dans ce cas la préparation dudit mélange à partir d'halogénure de benzyle nécessite deux étapes de nitration, ou bient ledit composé est obtenu par réaction d'halogénure de benzyle ou d'alcool benzylique sur du toluène ou d'halogénure de méthylbenzyle sur du benzène et dans ce cas ledit composé doit subir une opération de distillation avant nitration.

Le procédé objet de l'invention, qui part d'halogénure de benzyle, comporte une seule étape de nitration et, avant nitration, suivie d'hydrogénation et de phosgénation, du composé à structure diphénylméthane obtenu à partir d'halogénure de benzyle, il n'est pas nécessaire de réaliser une opération de distillation.

Selon le procédé de l'invention le composé à structure diphénylméthane est le diphénylméthane lui-même. Le diphénylméthane utilisé comme matière première peut être préparé par halogénation photochimique ménagée de toluène en halogénure de benzyle qu'on condense par réaction Friedel et Crafts sur un excès de benzène. Suivant les conditions opératoires et le catalyseur utilisé, en réglant l'excès de benzène, on obtient avec de bons rendements le diphénylméthane, mais il se forme aussi des produits de condensation à 3,4 noyaux et plus qui peuvent être séparés par une distillation. Cependant, on peut aussi nitrer le mélange brut, puis hydrogéner et phosgéner sans séparation préalable. On obtient donc ainsi un mélange de diisocyanates et de polyisocyanates, utilisable tel quel pour certaines applications de polyuréthannes, mais qui peut être distillé pour en extraire les isomères diarylés.

On utilise pour la nitration un mélange d'acide nitrique et d'acide sulfurique concentrés à, au moins 90% en poids. L'acide nitrique peut être placé en quantités stoechiométriques par rapport au composé aromatique utilisant ainsi une mole d'acide par noyau aromatique à nitrer. Il est cependant avantageux d'opérer en présence d'un excès d'acide nitrique pouvant aller jusqu'à 20% par rapport à la stoechiométrie. L'acide sulfurique peut être utilisé en quantité équimolaire par rapport à l'acide nitrique, mais on peut le placer en excès ou en défaut. La réaction de nitration peut s'effectuer entre 0°C et la température d'ébullition des mélanges, habituellement entre 0°C et 50°C, le composé aromatique étant de préférence solubilisé dans un solvant tel que le chlorure de méthylène. L'addition du mélange d'acides peut donc se faire à 0°C, mais il n'est pas gênant d'opérer à température ambiante à condition de contrôler l'exothermicité de la réaction. Celle-ci peut se poursuivre à température ambiante, mais il est préférable d'opérer au reflux du mélange, ce qui permet d'éliminer en partie l'excès d'acide nitrique. La phase organique, supérieure, est ensuite séparée des acides, neutralisée et évaporée à sec. Il est recommandé d'opérer sous atmosphère d'azote, dans un réacteur muni de moyens d'agitation efficace et de régulation de température.

En suivant le mode opératoire ainsi décrit, on obtient, à partir de diphénylméthane pur, des produits formés essentiellement d'isomères de dinitration, contenant de 32 à 55% de dinitro-4,4' diphénylméthane, de 30 à 45% d'isomère 2,4', de 5 à 12% d'isomère 3,4', de 4 à 10% d'isomère 2,2', de 2 à 5% d'isomère 2,3'. La nitration, dans des conditions identiques, d'un mélange de diphénylméthane et d'homologues supérieurs, conduit aux produits nitrés correspondants.

La deuxième étape constitué l'hydrogénation des dérivés nitrés en amines correspondantes; elle peut

2

**0 125 169**

être chimique, mais il est préférable d'opérer sous pression d'hydrogène, dans un réacteur résistant à la pression, muni d'une agitation et possédant les moyens de contrôle et régulation classiques, en présence d'un catalyseur à base de nickel, palladium, platine, ruthénium et autres.

Dans ce cas, on utilise un réacteur d'hydrogénation permettant de travailler sous des pressions pouvant attendre 100 bars. La réaction peut s'effectuer sans solvant à une température où le dérivé nitré est fondu, ou dans des solvants classiques d'hydrogénation tels que les alcools, le dioxane, les éthers de l'éthylèneglycol et autres.

On utilise préférentiellement un catalyseur constitué de palladium déposé sur support à des teneurs comprises entre 1 et 10%, ce qui permet d'opérer à des températures comprises entre 30 et 100°C et des pressions de 20 à 50 bars. Le rapport molaire

$$\frac{\text{dérivés nitrés}}{\text{palladium}}$$

n'est pas impérativement fixé, mais il est de préférence compris entre 200 et 3 000. Après réaction et filtration du catalyseur, le solvant éventuellement utilisé est évaporé et le mélange d'amines obtenu peut être utilisé tel quel. Il est aussi possible de séparer à ce stade les isomères de diamino-diphénylméthane par distillation. La troisième étape fait appel à des techniques classiques de phosgénation dans un réacteur muni d'une agitation et surmonté d'un réfrigérant. Par exemple, le mélange d'amines est placé à une concentration de 5 à 20% dans un solvant aromatique chloré tel que le monochlorobenzène ou l'orthodichlorobenzène contenant la quantité nécessaire de phosgène, en maintenant la température aux environs de 20°C. On chauffe ensuite la suspension progressivement. Le mélange réactionnel s'homogénéise vers 100°C. On continue d'élever lentement la température jusqu'au point d'ébullition du mélange, puis on distille le solvant de façon à récupérer les isocyanates formés. Ces derniers peuvent être utilisés tels quels par exemple dans certaines formulations pour polyuréthannes. Cependant, les isomères de diisocyanates de diphénylméthane peuvent être récupérés par distillation sous un vide poussé. Une autre technique consiste à introduire conjointement dans le réacteur une solution des isomères de diamine dans un solvant aromatique chloré et un courant de phosgène gazeux. La réaction peut être effectuée dans un premier temps à température ordinaire, puis le réacteur est progressivement chauffé comme précédemment; mais on peut aussi opérer directement aux environs de 100°C. La formation d'isocyanate est alors pratiquement immédiate.

Les isocyanates ainsi obtenus peuvent conduire, selon les techniques connues, à des polyuréthannes possédant de bonnes propriétés.

Les dérivés nitrés ont été analysés par chromatographie en phase gazeuse en utilisant une colonne en pyrex de diamètres intérieur et extérieur 3 et 6 mm, de longueur 2 mètres, remplie de phase SP 2250 imprégnée à 3% sur support Supelcoport 100—120 mesh (Supelco) sur laquelle on a effectué des programmations linéaires de température à 4°C/mn de 180°C à 280°C. Les isomères dinitrés principaux ont été synthétisés purs ou isolés par piégeage et identifiés par résonance magnétique nucléaire du [13]C et du [1]H.

Pour les amines, analysées également par chromatographie en phase gazeuse, on utilise une colonne en verre de 2 mètres, emplie de support chromosorb W.N.A.W. 60—80 mesh (Johns Manville), imprégnée à 5% de KOH et 5% d'Apiézon N (Société Apiézon Products Limited), en opérant en isotherme à 220°C. Les teneurs en fonctions aminées totales sont dosées chimiquement. Les fonctions NCO des isocyanates sont évaluées par dosage chimique, tandis que les compositions en isomères sont déterminées par chromatographie en phase gazeuse sur la colonne et dans les conditions utilisées pour les dérivés nitrés. Dans les cas où on opère sur les mélanges bruts contenant, outre du diphénylméthane, des homologues supérieurs, les dérivés nitrés, aminés, et phosgénés correspondants sont analysés en chromatographie par perméation de gel en utilisant une colonne Shodex A-802.

Exemple 1

On dissout 16,8 g de diphénylméthane pur (0,1 mole), préparé par condensation de Friedel et Crafts de chlorure de benzyle sur du benzène en excès et distillé, dans 40 ml de chlorure de méthylène, puis on ajoute goutte à goutte en une demi-heure, en maintenant la température en-dessous de 25°C, un mélange de 14,1 g d'acide nitrique à 98,5% et de 20 ml d'acide sulfurique à 96%. Dès la fin de l'addition, on porte à reflux pendant 3 H 30, puis refroidit. Deux phases se séparent, l'une inférieure acide, l'autre supérieure organique. On extrait la phase organique et lave deux fois les acides avec du chlorure de méthylène. On réunit les différentes phases organiques, les neutralise avec du carbonate de potassium et évapore le solvant. On récupère après broyage, 24,5 g d'une poudre jaune claire, de point de fusion 154°C contenant 99,3% d'isomères de dinitration de composition 48,3% de 4,4'; 33,2% de 2,4'; 8,2% de 3,4'; 6,3% de 2,2'; 3% de 2,3'.

On place 20 g de ce mélange d'isomères de dinitrodiphénylméthane dans l'autoclave et ajoute 0,2 litre de méthanol ainsi que 0,2 g de catalyseur constitué de palladium déposé sur charbon à 5%. Après avoir balayé le réacteur avec de l'azote, on y introduit 40 bars d'hydrogène et chauffe sous agitation jusqu'à 70°C. Après 1 heure de réaction, on laisse refroidir, décomprime et récupère le mélange réactionnel qu'on filtre

3

pour éliminer le catalyseur. Le méthanol est évaporé à sec et on obtient 15 g des isomères de dimaino-diphénylméthane. On dissout 14 g de ceux-ci dans 50 ml d'orthodichlorobenzène et introduit la solution, goutte à goutte par une ampoule de coulée, dans un réacteur contenant 38 g de phosgène dans un pied de 50 ml du même solvant, et maintenu à 20°C; on laisse réagir pendant une demi-heure à 20—25°C. Il se forme immédiatement un précipité blanc-gris. On chauffe ensuite à 45°C pendant 1 heure, puis 1 heure à 60°C et à 100°C pendant 1/2 heure. Le mélange s'homogénéise et on élève la température à 140°C pendant 2 heures, en faisant passer un courant d'azote afin de chasser le phosgène en excès et l'acide chlorhydrique formé. Puis, on évapore le solvant sous vide et recueille 17,5 g de mélange d'isocyanates dont la distillation conduit à un liquide jaune-clair constitué d'un mélange des isomères de diisocyanates de diphénylméthane (teneur en NCO: 2 équivalents par mole) de composition correspondant à l'objet de la présente invention: 44,5% de 4,4'; 36,7% de 2,4'; 7,7% de 3,4%; 7% de 2,2'; 3,2% de 2,3'.

Exemple 2 (comparatif)

16,8 g de diphénylméthane sont dissous dans 40 ml de chlorure de méthylène. On opère comme dans l'exemple 1, mais en utilisant 17 g d'acide nitrique à 85% et 30 g d'acide sulfurique à 85%. On laisse à reflux pendant 3 H 30, puis extrait et neutralise la phase organique. L'évaporation du solvant conduit à 22,5 g d'un produit jaune fondant à 136°C et contenant 7,7% de diphénylméthane n'ayant pas réagi, 1,3% de benzophénone résultant d'une oxydation, 17,2% d'isomères de mononitration et 72,4% des isomères attendus de dinitration. Il est évident qu'un tel mélange n'est plus intéressant dans le cadre de l'invention.

Exemple 3 (comparatif)

On prépare du dinitrodiphénylméthane suivant le mode opératoire décrit dans l'exemple 1, à partir de 201,6 g de diphénylméthane en utilisant 176 g d'acide nitrique à 98,7% et 240 ml d'acide sulfurique à 96%. On en prélève 40,7 g qu'on lave deux fois avec 200 ml d'éther afin d'éliminer totalement du mélange les isomères 2,2', et 2,3'; on récupère sur le filtre après séchage 24,2 g de précipité blanc de point de fusion 158°C et de composition: 68% de dinitro-4,4' diphénylméthane; 29% de dinitro-2,4' diphénylméthane et 3% de dinitro-3,4' diphénylméthane.

On hydrogène 20 g de ce mélange dans les conditions de l'exemple 1, ce qui conduit, après distillation du solvant, à 15,3 g de diamines se présentant sous une forme visqueuse de couleur orangée très claire.

La phosgénation de 15 g de ce produit donne, après distillation du mélange d'isomères, 16,3 g des diisocyanates dans la composition correspondant aux dinitrés (teneur en NCO: 2 équivalents par mole). Le point de fusion de ce mélange est alors de 33°C. Dans ce cas la propriété d'être liquide que possède le produit de l'invention contenant l'ensemble des isomères décrits dans la composition revendiquée est perdue.

Exemple 4

Dans 50 ml de chlorure de méthylène on dissout 20 g d'un mélange obtenu par condensation de Friedel et Crafts de chlorure de benzyle sur du benzène en présence de FeCl₃, ce mélange contenant 43% de diphénylméthane, 24% de dérivés triarylés, 15% de dérivés tétraarylés et 18% de composés à 5 noyaux et plus. On ajoute goutte à goutte en 30 minutes, en limitant la température en-dessous de 25°C, 16,7 g d'acide nitrique à 98,7% et 30,9 g d'acide sulfurique à 96%.

On laisse à reflux pendant 3 H 30, puis extrait et neutralise la phase organique. On obtient, après évaporation du solvant, 28,5 g d'un mélange des dérivés nitrés dont la teneur en azote est de 10,7% et la répartition en noyaux aromatiques analysés en chromatographie par perméation de gel est identique à celle du produit de départ. On hydrogène 17 g de ce mélange dans 0,2 l de méthanol, en présence de 0,2 g de palladium à 5% sur charbon. On récupère après filtration du catalyseur et évaporation du solvant, 13 g d'un mélange pâteux des amines qu'on peut couler à partir de 70°C.

On phosgène 10 g de ce mélange d'amines dans 100 ml d'orthodichlorobenzène en présence de 28 g de phosgène liquide en opérant de la façon décrite dans l'exemple 1. On récupère après filtration et évaporation du solvant 11,6 g d'un mélange liquide d'isocyanates (teneur en NCO: 6,86 fonctions par kilog.) contenant des isomères de diisocyanates de diphénylméthane de répartition: 49% de 4,4'; 34,1% de 2,4'; 7,8% de 3,4'; 6,1% de 2,2'; 2,7% de 2,3'.

Par ailleurs en distillant ce produit brut, pouvant cependant être utilisé tel quel, on obtient un mélange liquide des isomères diisocyanates de diphénylméthane dont la teneur en NCO est de 2 équivalents par mole de composition 40% de 4,4'; 42% de 2,4'; 5,7% de 3,4'; 8,7% de 2,2'; 3,2% de 2,3'.

Exemple 5

On utilise un mélange hydrocarboné résultant de la condensation de Friedel et Crafts de chlorure de benzyle sur le benzène, contenant 95% de diphénylméthane et 4,5% de dérivés triarylés. On en dissout 17,7 g dans 80 ml de chlorure de méthylène et ajoute goutte à goutte, tout en maintenant la température en dessous de 25°C, un mélange de 14,7 g d'acide nitrique à 98,7% et de 21,5 g d'acide sulfurique à 95%.

On laisse à reflux pendant 3 H 30, puis extrait et neutralise la phase organique. On obtient, après évaporation du solvant, 27 g d'un produit jaune clair légèrement pâteux contenant des isomères dinitrodiphénylméthane ayant la répartition: 44,2% de 4,4'; 36,5% de 2,4'; 8,8% de 2,2'; 7,9% de 3,4'; et 3,5% de 2,3'.

4

On hydrogène 20 g de ce mélange dans 200 ml de méthanol, en présence de 0,2 g de catalyseur au palladium à 5% sur charbon. On récupère, après filtration du catalyseur et évaporation du solvant, 15 g d'u mélange des amines.

On solubilise 10 g de ce mélange dans 50 ml d'orthodichlorobenzène, le filtre et l'introduit en 1 heure, dans un réacteur contenant 50 ml d'o.d.c.b. à 80°, en admettant conjointement un courant gazeux de phosgène de 2,5 l/h. On laisse réagir pendant 1 heure, puis monte à 100° pendant 1 heure puis à 140°, température à laquelle on coupe le phosgène pour balayer à l'azote pendant 2 heures. On distille le solvant et récupère 11,3 g d'un mélange liquide d'isocyanates (teneur en NCO: 7,7 équivalents par kg) dans lequel la répartition des isomères de diisocyanates de diphénylméthane est voisine de: 43,3% de 4,4'; 37,1% de 2,4'; 8,4% de 2,2'; 7,7% de 3,4'; 3,6% de 2,3'.

Exemple 6

On utilise un mélange hydrocarboné de condensation de Friedel et Crafts de chlorure de benzyle sur le benzène contenant 67% de diphénylméthane, 20% de dérivés triarylés, 9% de tétraarylés et 4% de pentaarylés et homologues supérieurs. On en dissout 20 g dans 80 ml de chlorure de méthylène et verse goutte à goutte, en maintenant la température en dessous de 25°C, un mélange de 16,8 g d'acide nitrique à 98,7% et de 31,3 g d'acide sulfurique à 96%.

On laisse à reflux pendant 3 H 30, puis extrait et neutralise la phase organique. On obtient, après évaporation du solvant, 30 g d'un mélange de dérivés nitrés de couleur marron et contenant des isomères de dinitrodiphénylméthane ayant la répartition: 48,2 de 4,4'; 33,6% de 2,4'; 8,4% de 3,4'; 6,5% de 2,2'; 3,2% de 2,3'.

On hydrogène 21 g de ce mélange dans 200 ml de méthanol, en présence de 0,2 g de catalyseur au palladium à 5% sur charbon. On récupère, après filtration du catalyseur et évaporation du solvant, 16,2 g du mélange des amines.

On solubilise 10 g de ce mélange dans 50 ml d'orthodichlorobenzène (o.d.c.b.), filtre et introduit en 1 heure, dans un réacteur contenant 50 ml d'o.d.c.b. à 80°C, en admettant conjointement un courant gazeux de phosgène de 3,1 l/h. En opérant comme dans l'exemple 5, on récupère 11,8 g d'un mélange liquide d'isocyanates (teneur en NCO: 7,69 équivalents par kg) dans lequel la répartition des isomères de diisocyanates de diphénylméthane est proche de 47,3 en 4,4'; 35% en 2,4'; 7,5% en 3,4'; 6,9% en 2,2'; 3,2% en 2,3'.

Exemple 7

On forme un prépolymère en faisant réagir à 60°C du MDI distillé préparé de la même façon que dans l'exemple 1 (teneur NCO: 2 équivalents/mole) avec un polypropylèneglycol de masse 2000 (référence Ugipol 1020) préalablement deshydraté. La quantité de MDI est telle que NCO/OH=2,05. Un test comparatif est effectué avec du MDI-4,4' commercial en paillettes:

|  | MDI de l'invention | MDI-4,4' commercial |
|---|---|---|
| Durée de réaction pour une conversion de 100% | 6 heures | 12 heures |
| Titre | 0,85 NCO/kg | 0,84 NCO/kg |
| Viscosité (25°C) en cps | 34 400 | 35 000 |
| Aspect | transparent | opaque |

Exemple 8

Des éprouvettes d'élastomères ont été synthétisées à partir de MDI distillé préparé de la même façon que dans l'exemple 1 (teneur en NCO: 8 éq./kg) et de MDI commercial liquide modifié partiellement par des carbodiimides et de titre 6,8 NCO/kg.

La mise au oeuvre sous forme de plaques coulées est faite à partir de polyadipate de butane diol (masse: 2000), de butane diol-1,4 et de MDI.

On mélange de polyadipate et le butanediol, préalablement déshydratés sous vide pendant 1 h à 80°C, puis ajoute le MDI à 40°C. Le mélange est dégazé sous vide et dès que la température atteint 55—60°C, l'élastomère en formation est coulé dans un moule. On cuit ~24 h à 100°C. Les résultats sont rassemblés dans le tableau ci-dessous:

**0 125 169**

| MDI | Conforme à l'invention | Commercial | Conforme à l'invention | Commercial |
|---|---|---|---|---|
| Teneur en butane diol-1,4 (%en poids) | 10 | 10 | 20 | 20 |
| Aspect de l'élastomère | translucide | opaque | transparent | opaque |
| Dureté Shore A | 77 | 82 | 62 | 89 |
| Résistance à la traction Charge de rupture (kgf/cm$^2$) | 339 | 377 | 424 | 480 |
| Allongement (%) | 446 | 407 | 642 | 275 |

**Revendications**

1. Composition liquide contenant des diisocyanates de structure diphénylméthane dont la répartition en poids est de:

32 à 55% d'isomère 4,4'
30 à 45% d'isomère 2,4'
5 à 12% d'isomère 3,4'
4 à 10% d'isomère 2,2'
2 à 5% d'isomère 2,3'

2. Procédé de fabrication de la composition selon la revendication 1 caractérisé en ce qu'elle est obtenue par réaction initiale d'un halogénure de benzyle sur le benzène suivie d'une nitration des noyaux aromatiques, d'une hydrogénation et d'une phosgénation.

3. Procédé de fabrication selon la revendication 2 caractérisé en ce que la nitration s'effectue au moyen d'un mélange d'acide nitrique et d'acide sulfurique de concentrations au moins égales à 90%.

**Patentansprüche**

1. Flüssiges Mittel enthaltend Diisocyanate mit Diphenylmethanstruktur, deren gewichtsmäßig Verteilung lautet:

32—55% 4,4'-Isomer
30—45% 2,4'-Isomer
5—12% 3,4'-Isomer
4—10% 2,2'-Isomer
2— 5% 2,3'-Isomer.

2. Verfahren zur Herstellung des Mittels nach Anspruch 1, dadurch gekennzeichnet, daß es durch zunächst Einwirkung eines Benzylhalogenids auf Benzol und anschließende Nitrierung der aromatischen Kerne, Hydrierung und Phosgenierung erhalten wird.

3. Herstellungsverfahren nach Anspruch 2, dadurch gekennzeichnet, daß die Nitrierung mit Hilfe eines Gemisches aus Salpetersäure und Schwefelsäure in Konzentrationen von mindestens 90% erfolgt.

**Claims**

1. Liquid composition containing diisocyanates having a diphenylmethane structure, the distribution by weight of which is as follows:

32 to 55% of 4,4' isomer
30 to 45% of 2,4' isomer
5 to 12% of 3,4' isomer
4 to 10% of 2,2' isomer
2 to 5% of 2,3' isomer.

2. Process for manufacturing the composition according to Claim 1, characterized in that it is obtained

6

by initially reacting a benzyl halide with benzene, followed by nitration of the aromatic rings, hydrogenation and phosgenation.

3. Manufacturing process according to Claim 2, characterized in that the nitration is performed by means of a mixture of nitric acid and sulphuric acid having concentrations equal to at least 90%.